# EUROPÄISCHE PATENTSCHRIFT

(11) **EP 4 007 616 B1**
(45) Veröffentlichungstag und Bekanntmachung des Hinweises auf die Patenterteilung: **07.05.2025**
(21) Anmeldenummer: 20746969.3
(22) Anmeldetag: 28.07.2020
(51) Int. Cl.: A61M 1/14, A61M 1/34, A61M 1/36

(54) **EXTRAKORPORALE-BLUTBEHANDLUNSSYSTEM MIT AUGMENTED-REALITY VORRICHTUNG ZUR ÜBERPRÜFUNG DER MONTAGE UND MIT FUNKTIONSTEST**
EXTRACORPOREAL BLOOD TREATMENT SYSTEM WITH AUGMENTED-REALITY DEVICE FOR CHECKING THE ASSEMBLAGE AND WITH FUNCTION TEST
SYSTÈME DE TRAITEMENT EXTRACORPOREL DU SANG AVEC UN DISPOSITIF À RÉALITÉ AUGMENTÉE POUR VÉRIFIER L'ASSEMBLAGE ET AVEC TEST DE FONCTIONNEMENT

(30) Priorität: 02.08.2019 DE 102019120999
(43) Veröffentlichungstag der Anmeldung: 08.06.2022
(73) Patentinhaber: B. Braun Avitum AG, 34212 Melsungen (DE)
(72) Erfinder: BAUER, Florian, 34212 Melsungen (DE); SCHLEICHER, Christian, 36160 Dipperz (DE)
(74) Vertreter: Winter, Brandl - Partnerschaft mbB
(86) Internationale Anmeldenummer: PCT/EP2020/071234
(87) Internationale Veröffentlichungsnummer: WO 2021/023574

(56) Entgegenhaltungen:
- US-A1- 2019 064 520

## Beschreibung

### Technisches Gebiet

Die Offenbarung betrifft ein Verfahren und eine Vorrichtung zur Funktionsprüfung von medizinischen Geräten.

### Hintergrund der Offenbarung

Unter erweiterter Realität (auch englisch augmented reality, kurz AR) versteht man die computergestützte Erweiterung der Realitätswahrnehmung. Diese Information kann alle menschlichen Sinnesmodalitäten ansprechen. Häufig wird jedoch unter erweiterter Realität nur die visuelle Darstellung von Informationen verstanden, also die Ergänzung von Bildern oder Videos mit computergenerierten Zusatzinformationen oder virtuellen Objekten mittels Einblendung/Überlagerung. Bei Fußball-Übertragungen ist erweiterte Realität beispielsweise das Einblenden von Entfernungen bei Freistößen mithilfe eines Kreises oder einer Linie.

Ein Dialysegerät/ eine Dialysemaschine ermöglicht die patientenspezifische Entfernung gelöster Substanzen (z. B. Harnstoff, Kreatinin, Vitamin B12 oder β2-Mikroglobulin) sowie gegebenenfalls eines definierten Wasseranteils aus dem Blut bei Nierenersatzbehandlungen. Dialysegeräte werden sowohl für die Hämodialyse als auch die Hämodiafiltration eingesetzt. Grundsätzlich kann man Dialysegeräte in folgende Module einteilen: Extrakorporaler Blutkreislauf, Dialysierflüssigkeitskreislauf, Desinfektionseinheit, Bedienteil und Netzteil. Darüber hinaus kommen Verbrauchsmaterialien, sogenannte Disposables, während der Behandlung zum Einsatz. Diese Disposables sind zum Beispiel Dialysierflüssigkeitskanülen, Blutschlauchsysteme, Dialysatoren, Dialysekonzentrate usw.

Ein Nachteil im Stand der Technik ist es, dass Pflegekräfte ein spezielles Anwendertraining benötigen, um Dialysegeräte zu benutzen bzw. die Disposables richtig an das Dialysegerät anzubringen. Dieses Anwendertraining ist für jede neue Generation einer Dialysemaschine notwendig.

Aus der US 2019/0064520 A1 ist eine Augmented-Reality-Unterstützung während einer Dialysebehandlung bekannt.

Beschreibung der Erfindung Die Aufgabe der Offenbarung ist es, ein schnelles, effizientes und fehlerfreies Anlegen der Disposables sowie einen schnellen und sicheren Selbsttest durch interne und externe Informationen zu ermöglichen.

Gelöst wird diese Aufgabe durch die Merkmale der Ansprüche 1 und 7. Vorteilhafte Ausführungsformen bzw. Weiterbildungen sind in den Unteransprüchen beansprucht Neben dieser beanspruchten Erfindung beschreibt das vorliegende Dokument auf allgemeinere Weise (ohne es jedoch auf diese allgemeinere Weise zu beanspruchen) : ein Verfahren zur Aktivierung einer Funktion einer Vorrichtung, vorzugsweise medizinischen Vorrichtung, aufweisend die Schritte:
- Erfassen des Ist-Zustandes der Vorrichtung mittels einer Kamera vorzugsweise an einem AR-Device, insbesondere einer AR-Brille oder einer AR-Sichtschutzscheibe,
- Vergleichen des Ist-Zustandes der Vorrichtung mit einem Soll-Zustand der Vorrichtung durch wenigstens eine CPU, die mit der Kamera in Verbindung steht,
- Senden eines Befehls für den Start einer Funktion, vorzugsweise eines Funktionstests, von der CPU an die Vorrichtung, auf Basis des Vergleichs,
- Aktivieren einer Funktion, vorzugsweise eines Funktionstests, der Vorrichtung, aufgrund des Befehls.

In anderen Worten ausgedrückt erkennt die wenigstens eine CPU/ Recheneinheit, die die Bilder von der Kamera erhält, den Zustand/ Aufbau/ Setting/ Montagestufe einer medizinischen Vorrichtung, vorzugsweise einer Dialysemaschine. Dabei können die Kamera und gegebenenfalls auch die CPU/Recheneinheit in dem AR-Device, vorzugsweise an einer Smart-Glass-Brille untergebracht bzw. in diese integriert sein.

Die wenigstens eine CPU/ Recheneinheit blendet die vom Anwender durchzuführenden erforderlichen Schritte z.B. zum korrekten Verbinden von Disposables mit der medizinischen Vorrichtung in ein Sichtfeld des AR-Device insbesondere einer Smart-Glas-Brille ein (vorzugsweise blendet ein Projektor die Informationen ein, der mit der CPU in Verbindung steht).

Wenn die CPU über die an dem AR-Device angeordnete Kamera erkennt, dass ein Aufbau richtig beendet ist, startet die CPU über eine vorzugsweise kabellose Verbindung vorzugsweise mittels Bluetooth oder WLAN (ZigBee, NFC, Mobilfunk) die Vorrichtung zu einem Funktionstest.

Alternativ oder zusätzlich ist die CPU oder eine Datenübertragungsvorrichtung an dem AR-Device dafür vorgesehen und angepasst die Bilder der Kamera an eine andere, zweite bzw. stationäre CPU zu senden. Vorzugsweise befindet sich diese in einer/ der medizinischen Vorrichtung, sie kann aber auch extern von dem AR-Device und/oder der medizinischen Vorrichtung sein, z.B. in Form eines PCs der mit der medizinischen Vorrichtung verbunden ist. Diese zweite bzw. stationäre CPU hat vorzugsweise eine größere Rechenleistung als die erste CPU, die gegebenenfalls an dem AR-Device angeordnet wird/ ist. Die zweite CPU verarbeitet die empfangenen Daten und sendet verarbeitete Daten wieder an die erste CPU der Datenübertragungsvorrichtung an das AR-Device, das dann veranlasst, dass die Informationen in dem Sichtfeld des AR-Devices angezeigt werden. In anderen Worten ausgedrückt dient die erste CPU an dem AR-Device zur Übertragung der Daten und steuert die Anzeige von Informationen, wohingegen die zweite externe bzw. stationäre CPU als Datenverarbeitungseinheit sowie Recheneinheit zum Verarbeiten der aufgenommenen Bilder dient und daraus z.B. Handlungsschritte ableitet, die sie wie eben beschrieben an die erste CPU als konkrete anzuzeigende Informationen zurückgibt.

Vorzugsweise ist die Vorrichtung eine medizinische Vorrichtung und besonders bevorzugt ein Dialysegerät, eine Dialysemaschine, eine Spritzenpumpe oder dergleichen.

Vorzugsweise ist das AR-Device ein Produkt der Klasse der Wearables (Wearable Computing). Ein Wearable/ Wearable Computer ist während der Anwendung am Körper des Benutzers befestigt oder in die Kleidung integriert. Vorzugsweise weist das AR-Device gemäß der Offenbarung ein optisches Display auf, das in dem Sichtfeld eines Anwenders integriert ist, sowie eine CPU und/oder eine Datenübermittlungseinheit. Das AR- Device ist besonders bevorzugt eine AR-Brille, Smart-Glass-Brille, Smartglasses, eine Datenbrille oder ein Head-up-Display. Eine Smart-Glass-Brille oder Smartglasses (oder umgangssprachlich: Datenbrille) ist dabei vorzugsweise ein tragbarer (wearable) Computer an einer Brille, der Informationen zum Sichtfeld des Benutzers hinzufügt. Weiter vorzugsweise weist die Brille auch die Kamera auf. Zusätzlich oder alternativ kann die Brille auch eine Datenübertragungseinheit aufweisen. Die Brille ermöglicht augmented Reality bzw. mixed Reality.

Vorzugsweise ist die Kamera eine Digitalkamera. Neben der diskreten Anzeige von Informationen auf dem Display des AR-Devices können Informationen auch mit dem aufgenommenen Bild der integrierten Digitalkamera kombiniert werden. Dazu können Daten aus dem Internet unmittelbar bezogen und versendet werden.

Vorzugsweise ist die Kamera mit der ersten (AR-Device integrierten) CPU und/ oder der zweiten stationären bzw. separaten CPU in datentechnischer Verbindung, vorzugsweise drahtlos, z.B. über Bluetooth, W-LAN oder dergleichen. Alternativ oder zusätzlich kann die erste CPU an dem AR-Device mit einer zweiten externen CPU in datentechnischer Verbindung, vorzugsweise drahtlos, z.B. über Bluetooth, W-LAN oder dergleichen stehen. Vorzugsweise ist die CPU an dem AR-Device mit der medizinischen Vorrichtung in datentechnischer Verbindung, vorzugsweise drahtlos, z.B. über Bluetooth, W-LAN oder dergleichen. Bei der zweiten CPU handelt es sich bevorzugt um eine in der medizinischen Vorrichtung zu Steuerungszwecken derselben bereits vorhandene Recheneinheit/ CPU.

Vorzugsweise findet der Vergleich des Ist-Zustands der Vorrichtung mit einem Soll-Zustand der Vorrichtung mittels eines Bilderkennungsalgorithmus statt, der auf einem Speichermedium der CPU gespeichert ist. Wobei dies die erste CPU an dem AR-Device sein kann, oder wenigstens eine optionale zweite CPU in der medizinischen Vorrichtung oder extern von dem AR-Device und/ oder der medizinischen Vorrichtung.

Vorzugsweise führt die Vorrichtung einen Selbsttest/ Funktionstest/ Druckselbsttest oder dergleichen in Erwiderung auf den Input der Kamera durch. Dabei kann der Funktionstest nach einer manuellen Bestätigung an der medizinischen Vorrichtung selbst, oder mittels einer Geste, die von der Kamera und der CPU erkannt wird, oder einfach nach einer vorgegebenen Zeit aktiviert werden. Beispielsweise kann nach Anschluss der Drucksensoren ein Druckselbsttest gestartet werden. Bei dem Druckselbsttest werden vorzugsweise die angeschlossenen/ montierten Disposables an der medizinischen Vorrichtung von dieser unter Druck gesetzt. Falls ein vorbestimmter Druckabfall von Drucksensoren ermittelt wird, liegt eine Leckage bzw. ein fehlerhafter Anschluss vor und die medizinische Vorrichtung gibt eine Fehlermeldung/ Signal aus.

Der Ist-Zustand bezeichnet den aktuellen Zustand inwieweit die Disposables in die medizinische Vorrichtung eingebaut sind zu einem ersten Zeitpunkt T1. Der Soll-Zustand bezeichnet den fertigen und richtigen Zustand der Disposables in der medizinischen Vorrichtung. Vorzugsweise wird der Aufrüstvorgang, bei dem die Disposables in die medizinische Vorrichtung eingebaut werden, in verschiedene Teilschritte zerlegt und diese werden, vorzugsweise jeweils einzeln oder im Gesamten, angeleitet, überwacht (Vergleich zwischen aktuellem Ist-Zustand und Soll-Zustand des Teilschrittes) und dann ,getestet'.

Das Senden eines Befehls für den Start einer Funktion, vorzugsweise eines Funktionstests, von der CPU an die Vorrichtung, auf Basis des Vergleichs (bzw. im Anschluss an den Vergleich) geschieht vorzugsweise bei einem positiven Vergleich, also wenn der Ist-Zustand dem Soll-Zustand entspricht. Bei einem negativen Vergleich, also wenn der Ist-Zustand nicht dem Soll-Zustand entspricht, wird auf den Befehl hin eine entsprechende Funktion aktiviert, vorzugsweise eine Fehlermeldung in dem AR-Device oder der medizinischen Vorrichtung.

Vorzugsweise ist das Verfahren dadurch gekennzeichnet, dass es des Weiteren den Schritt aufweist: Anzeigen des Funktionsergebnisses in dem AR-Device. In anderen Worten ausgedrückt werden die einzelnen Einbauschritte der Disposables in dem AR-Device angezeigt, in Abhängigkeit vom Vergleich des Ist-Zustands mit dem Soll-Zustand. Alternativ oder zusätzlich kann ein Ergebnis des Funktionstests, zum Beispiel der vorherrschende Druck in den angeschlossenen Disposables angezeigt werden.

Vorzugsweise ist das Verfahren dadurch gekennzeichnet, dass das Verfahren des Weiteren den Schritt aufweist: Senden eines Funktionstestergebnisses an die CPU und/oder Einblenden der erforderlichen Schritte, vorzugsweise in Form von Markierungen, Animationen und/ oder Nachrichten, zum Erreichen des Soll-Zustandes der Vorrichtung in dem AR-Device. In anderen Worten ausgedrückt sendet die medizinische Vorrichtung, in der zum Beispiel die zweite bzw. separate bzw. stationäre CPU und/ oder eine Datenübertragungseinheit vorhanden ist ein Signal an die erste CPU oder Datenübertragungseinheit in dem AR-Device. Analog dazu wird dann, wie vorstehend bereits erläutert, in Erwiderung auf das Signal der medizinischen Vorrichtung, das Ergebnis der Funktion (Funktionsergebnis/ Funktionsresultat) angezeigt.

Vorzugsweise ist das Verfahren dadurch gekennzeichnet, dass es des Weiteren den Schritt aufweist: Sperren weiterer Funktionen bei einer negativen Funktion (negativ ausgefallenem Funktionstest). In anderen Worten ausgedrückt, wenn der Ist-Zustand nicht mit dem Soll-Zustand übereinstimmt, sperrt die medizinische Vorrichtung weitere Funktionen, bis der Vergleich positiv ist.

Vorzugsweise ist das Verfahren dadurch gekennzeichnet, dass ein Funktionstest ein Drucktest ist, der einen zu Testzwecken aufgebauten (Leitungs-) Druck in Disposables misst.

Vorzugsweise werden die Schritte graphisch auf einem Display dargestellt. Bevorzugt werden in dem AR-Device die erforderlichen Schritte mittels bildlicher Darstellungen, die ganz oder teilweise animiert sein können und/oder mittels Schrift im Sichtfeld des Anwenders dargestellt. In anderen Worten ausgedrückt können Markierungen, Animationen und/oder Nachrichten in das Sichtfeld projiziert werden.

Vorzugsweise ist die medizinische Vorrichtung in datentechnischem Kontakt mit der CPU, die sich in dem AR-Device, der medizinischen Vorrichtung, einem PC oder anderweitig extern befinden kann.

Die Offenbarung betrifft des Weiteren ein System zur Aktivierung einer Vorrichtungs-Funktion, vorzugsweise eines Funktionstests, aufweisend wenigstens eine Kamera, wenigstens eine CPU die in datentechnischer Verbindung mit der Kamera steht, und vorzugsweise wenigstens eine Vorrichtung, beispielsweise eine medizinische Vorrichtung, insbesondere eine Dialysemaschine, die wiederum in datentechnischer Verbindung mit der CPU steht oder bringbar ist, wobei die CPU ein Speichermedium aufweist oder mit einer externen CPU datentechnisch verbindbar ist, auf dem/ der die folgenden Schritte gespeichert sind:
- Erkennen eines Ist-Zustandes der Vorrichtung mittels der Kamera, die vorzugsweise an einem AR-Device angeordnet ist,
- Vergleichen des Ist-Zustandes der Vorrichtung mit einem Soll-Zustand der Vorrichtung durch die CPU, die mit der Kamera in Verbindung steht,
- Senden eines Befehls für den Start einer Vorrichtungs-Funktion, vorzugsweise eines Vorrichtungs-Funktionstests, (von der CPU) an die Vorrichtung, wenn der Ist-Zustand einem vorgegebenen Soll-Zustand entspricht,
- Aktivieren der Vorrichtungs-Funktion, vorzugsweise des Vorrichtungs-Funktionstests, der Vorrichtung, aufgrund des Befehls.

Vorzugsweise ist die Kamera an/ in einem AR-Device angebracht, das vorgesehen und angepasst ist, die Ergebnisse des Funktionstests und/oder eine Einbauanleitung, vorzugsweise nach dem Anzeigeprinzip eines Head-UP-Displays, darzustellen.

Die Offenbarung betrifft des Weiteren vorzugsweise ein Verfahren und eine Vorrichtung zur Aktivierung einer Funktion einer Vorrichtung, vorzugsweise medizinischen Vorrichtung, aufweisend die Schritte: Erkennen des Ist-Zustandes der Vorrichtung mittels einer Kamera, die vorzugsweise an einem AR-Device angeordnet ist. Vergleichen des Ist-Zustandes der Vorrichtung mit einem Soll-Zustand der Vorrichtung durch eine CPU, die mit der Kamera in Verbindung steht. Senden eines Befehls für den Start einer Vorrichtungs-Funktion, vorzugsweise eines Vorrichtungs-Funktionstests, (von der CPU) an die Vorrichtung, wenn der Ist-Zustand gemäß visueller Erkennung einem vorgegeben Soll-Zustand entspricht bzw. zu entsprechen scheint. Aktivieren einer Vorrichtungs-Funktion, vorzugsweise eines Vorrichtungs-Funktionstests, in Ansprechen(Erwiderung) auf den Befehl.

Zusammengefasst betrifft die Offenbarung ein Verfahren zur Aktivierung oder Durchführung eines Funktionstests einer Blutbehandlungsvorrichtung, vorzugsweise Dialysemaschine, aufweisend die Schritte: Erfassen eines Ist-Zustandes der Blutbehandlungsvorrichtung mittels einer Kamera, welche vorzugsweise in/an einer Augmented-Reality-Vorrichtung, vorzugsweise einer Augmented-Reality-Brille, vorgesehen bzw. angebracht ist; Vergleichen des Ist-Zustandes der Blutbehandlungsvorrichtung mit einem Soll-Zustand der Blutbehandlungsvorrichtung durch eine Steuereinheit (CPU), die mit der Kamera in Verbindung steht; Senden eines Befehls für den Start des Funktionstests von der Steuereinheit (CPU) an die Blutbehandlungsvorrichtung auf Basis des Vergleichs; und Aktivieren des Funktionstests der Blutbehandlungsvorrichtung aufgrund des Befehls.

Bevorzugt weist das Verfahren ferner die Schritte auf: Senden eines Funktionstestergebnisses an die Steuereinheit (CPU); und Anzeigen des Funktionstestergebnisses in einer Augmented-Reality-Vorrichtung.

Weiter bevorzugt weist das Verfahren ferner den Schritt auf: Einblenden von noch erforderlichen durchzuführenden Schritten zum Erreichen des Soll-Zustandes der Blutbehandlungsvorrichtung in der Augmented-Reality-Vorrichtung, vorzugsweise in Form von Markierungen, Animationen und/ oder Nachrichten.

Es ist von Vorteil, wenn das Verfahren weiterhin den Schritt aufweist: Sperren weiterer Funktionstests bei einem negativen Funktionstestergebnis.

In vorteilhafter Weise ist der Funktionstest ein, vorzugsweise automatisierter, Selbsttest der Blutbehandlungsvorrichtung zum Überprüfen von von einem Nutzer vorgenommenen Aufrüstungshandlungen bei einer Aufrüstung der Blutbehandlungsvorrichtung.

Bevorzugt ist der Funktionstest ein Drucktest, der einen zu Testzwecken temporär aufgebauten Druck in Einmalprodukten, insbesondere Fluidleitungen, welche an/in der Blutbehandlungsvorrichtung angebracht bzw. vorgesehen sind, misst.

Weiterhin betrifft die Offenbarung zusammengefasst ein System aufweisend wenigstens eine Kamera, wenigstens eine Steuereinheit (CPU), die in datentechnischer Verbindung mit der Kamera steht oder bringbar ist, und wenigstens eine Blutbehandlungsvorrichtung, insbesondere Dialysemaschine, die wiederum in datentechnischer Verbindung mit der Steuereinheit (CPU) steht oder bringbar ist, wobei die Steuereinheit (CPU) eingerichtet ist, einen Ist-Zustand der Blutbehandlungsvorrichtung (8) mittels der Kamera (4), die vorzugsweise in/an einer Augmented-Reality-Vorrichtung (2), insbesondere einer Augmented-Reality-Brille oder einer Augmented-Reality-Sichtschutzscheibe, vorgesehen bzw. angeordnet ist, zu erfassen, den Ist-Zustand der Blutbehandlungsvorrichtung (8) mit einem Soll-Zustand der Blutbehandlungsvorrichtung (8) zu vergleichen, einen Befehl für den Start eines Funktionstests an die Blutbehandlungsvorrichtung (8) auf der Basis des Vergleichs zu senden, und den Funktionstest der Blutbehandlungsvorrichtung (8) aufgrund des Befehls zu aktivieren.

Bevorzugt ist die Kamera an/in einer Augmented-Reality-Vorrichtung angebracht bzw. vorgesehen, wobei die Augmented-Reality-Vorrichtung eingerichtet ist, ein Funktionstestergebnis und/oder weitere von einem Anwender durchzuführende Schritte darzustellen.

Weiter bevorzugt ist der Funktionstest ein, vorzugsweise automatisierter, Selbsttest der Blutbehandlungsvorrichtung zum Überprüfen von von einem Nutzer vorgenommenen Aufrüstungshandlungen bei einer Aufrüstung der Blutbehandlungsvorrichtung.

Es ist von Vorteil, wenn der Funktionstest ein Drucktest ist, der einen zu Testzwecken temporär aufgebauten Druck in Einmalprodukten, insbesondere Fluidleitungen, welche an/in der Blutbehandlungsvorrichtung angebracht bzw. vorgesehen sind, misst.

Im Nachfolgenden wird die vorliegende Offenbarung anhand eines bevorzugten Ausführungsbeispiels unter Bezugnahme auf die begleitenden Figuren näher erläutert.

### Kurzbeschreibung der Figuren

Figur 1 zeigt das System zur Aktivierung einer Funktion einer Vorrichtung gemäß der Offenbarung in einer schematischen Darstellung.
Figur 2 zeigt das Verfahren zur Aktivierung einer Funktion einer Vorrichtung.
Figur 3 zeigt das System zur Aktivierung einer Funktion einer Vorrichtung gemäß der Offenbarung in einer schematischen Darstellung mit einem augmented Reality Guide.
Figur 4 zeigt eine schematische Darstellung einer möglichen Interaktion zwischen dem AR-Device, dem Anwender und der CPU.

### Beschreibung des Ausführungsbeispiels

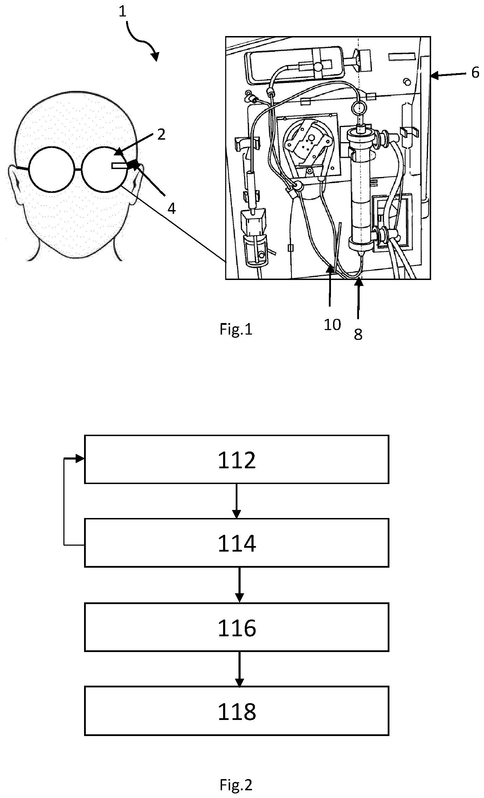

Figur 1 zeigt ein System 1 zur Aktivierung einer Funktion oder eines Funktionstests einer medizinischen Vorrichtung gemäß der Offenbarung in einer schematischen Darstellung. An/ in einer AR-Brille (AR-Device) 2 ist eine Kamera 4 angebracht, die ein definiertes Sichtfeld 6 umfasst. Das Sichtfeld 6 umfasst z.B. eine Dialysemaschine 8, als medizinische Vorrichtung, mit daran befestigten Disposables 10, die in diesem Fall die zum Auswechseln bestimmten Leitungen (Blutleitungen, Dialysierflüssigkeitsleitungen) und ein Dialysator der Dialysemaschine 8 sind. Des Weiteren ist wenigstens eine CPU vorgesehen, die mit der Kamera 4 in Verbindung steht. Diese CPU kann eine stationäre, gegebenenfalls Vorrichtungs-eigene Recheneinheit oder eine mobile AR-Brille-eigene Recheneinheit sein, wobei im Fall einer stationären Recheneinheit die AR-Brille lediglich eine Datenübertragungseinheit aufweisen kann. Die CPU (nicht dargestellt) vorzugsweise in der Dialysemaschine 8 und/oder an der AR-Brille 2 erkennt die Ist-Anordnung (Ist-Zustand) der Disposables 10 und vergleicht sie mittels eines Bilderkennungsalgorithmus mit einer Soll-Anordnung (Soll-Zustand), welche in einem Speichermedium der CPU abgespeichert ist/ oder der per W-LAN abgerufen wird. Erkennt der Algorithmus, dass die Disposables 10 falsch/ unvollständig angebracht sind, wird ein Korrekturschritt im Sichtfeld des Anwenders, das dem Sichtfeld 6 der Kamera 4 entspricht, in der AR-Brille 2 eingeblendet. Erkennt der Algorithmus, dass die Disposables 10 richtig angebracht sind, gibt die CPU vorzugsweise mittels W-LAN oder Bluetooth oder dergleichen einen Befehl an die Dialysemaschine 8 zur Durchführung einer Funktion oder eines Funktionstests. Ist der Funktionstest positiv, wird dies auf der AR-Brille 2 angezeigt. Ist der Funktionstest negativ, wird ein entsprechend erforderlicher Korrekturschritt auf der AR-Brille 2 angezeigt.

Figur 2 zeigt das Verfahren zur Aktivierung einer Funktion/ eines Funktionstests einer Vorrichtung (Dialysemaschine 8) unter Einbindung des vorstehenden Systems 1.

In Schritt 112 erkennt die Kamera 4 den Ist-Zustand der Dialysemaschine 8. In anderen Worten ausgedrückt nimmt die Kamera 4 ein oder mehrere Bilder auf, das/ die den aktuellen Ist-Zustand darstellt/ dokumentieren.

In Schritt 114 vergleicht die CPU den Ist-Zustand der Dialysemaschine 8 mit einem Soll-Zustand der Dialysemaschine 8. In anderen Worten ausgedrückt wird das Bild der Kamera 4 aus dem Schritt 112 mit einem gespeicherten Bild auf der CPU verglichen und mittels Mustererkennung bestimmt, ob der Ist-Zustand dem Soll-Zustand entspricht. Ist ein solcher Vergleich nicht möglich, etwa aufgrund eines ungeeigneten Bildes, wird der Schritt 112 erneut ausgeführt, bis ein Bild erhalten wird, das den Ist-Zustand zeigt und mit einem Soll-Zustand verglichen werden kann. Dieser Wiederholungsvorgang bzw. das Vorliegen eines ungeeigneten Bildes kann auf dem AR-Device angezeigt werden etwa mit einer bevorzugten Nachricht/ Korrekturanweisung, die zum Beispiel angibt, dass der Blickwinkel geändert werden muss. Kommt ein Vergleich des Ist-Zustandes mit Soll-Zustand zustande, folgt der nächste Schritt.

In Schritt 116 sendet die CPU einen Befehl zum Start eines Funktionstests an die Dialysemaschine 8. In anderen Worten ausgedrückt, sobald die CPU den Ist-Zustand mit dem Soll-Zustand positiv verglichen und festgestellt hat, ergeht ein Befehl an die medizinische Vorrichtung/ Dialysemaschine zur Ausführung einer Funktion/ eines Funktionstests zur Überprüfung, ob die Montage funktional korrekt ausgeführt wurde.

In Schritt 118 führt die Dialysemaschine 8 die Funktion bzw. den Funktionstest durch. In anderen Worten ausgedrückt erfolgt in Erwiderung auf den Befehl der CPU eine entsprechende Aktion an der Dialysemaschine 8.

In Schritt 120 sendet die Dialysemaschine 8 das Ergebnis des Funktionstests an die CPU bzw. misst die CPU das Testergebnis aus, die dann das Ergebnis graphisch auf der AR-Brille 2 darstellt. Vorzugsweise ist das Ergebnis dabei der Druck/ Druckverlauf, der in den Disposables/ Leitungen herrscht/ testweise induziert wurde.

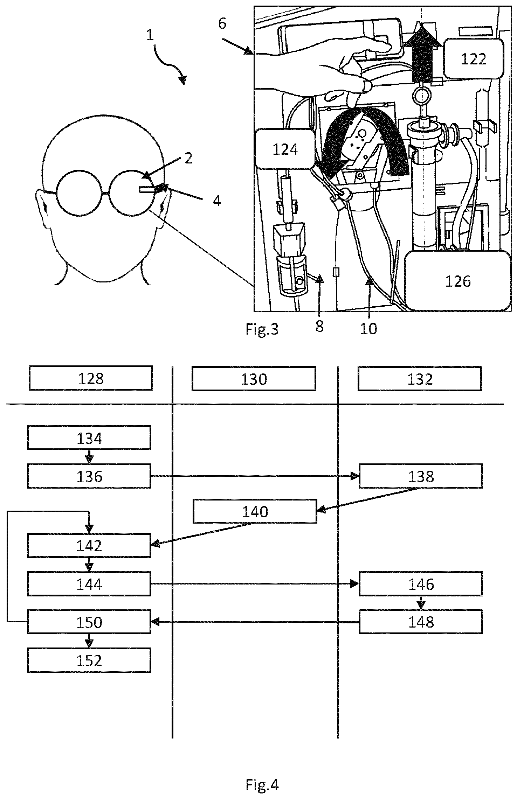

Figur 3 zeigt das System 1 zur Aktivierung einer Funktion bzw. eines Funktionstests einer medizinischen Vorrichtung gemäß der Offenbarung in einer schematischen Darstellung mit einem augmented Reality Guide in Anlehnung an Figur 2. An der AR-Brille 2 ist die Kamera 4 angebracht, die das definierte Sichtfeld 6 umfasst. Das Sichtfeld 6 (entspricht der Darstellung in Figur 3) umfasst z.B. die Dialysemaschine 8 (medizinische Vorrichtung) mit den daran befestigten Disposables 10 (z.B. Fluidleitungen, Schläuche, Dialysator, etc.). In dem Sichtfeld werden die Fenster 122, 124 und 126 eingeblendet und Pfeile, die Instruktionen für die Handhabung darstellen (z.B. der gerade Pfeil neben Fenster 122 und der halbrunde Pfeil neben Fenster 124). Das erste Fenster 122 kann Instruktionen enthalten wie zum Beispiel "Führen des Schlauchs durch den Verbinder". Das zweite Fenster 124 kann Instruktionen enthalten wie zum Beispiel "Einführen des Blutschlauches in die Blutpumpe wie in dem Video gezeigt". Das dritte Fenster 126 an dem unteren Rand kann eine Größe aufweisen, die unterschiedlich zu einem der anderen Fenster ist, zum Beispiel größer, und in diesem Fenster kann ein Video gezeigt werden, zum Beispiel ein Youtube-Video.

Figur 4 zeigt eine schematische Darstellung einer möglichen Interaktion zwischen dem AR-Device, dem Anwender und der CPU. In Figur 4 ist das Verfahren aufgeteilt in die Verfahrensschritte, die das AR-Device durchführt (siehe Spalte 128), in die Verfahrensschritte, die der Anwender durchführt (siehe Spalte 130) und in die Verfahrensschritte, die die medizinische Vorrichtung durchführt (siehe Spalte 132).

In Schritt 134 wird von dem AR-Device bzw. der daran befestigten CPU erkannt, dass die Aufgabe Verbinden der Disposables erkannt wurde.

In Schritt 136 wird die medizinische Vorrichtung informiert, dass die Aufgabe Verbinden der Disposables erkannt wurde.

In Schritt 138 bereitet sich die medizinische Vorrichtung auf einen Funktionstest vor.

In Schritt 140 werden dann vom Anwender die Leitungen eingebaut.

In Schritt 142 erkennt das AR-Device, dass die Leitungen angeschlossen wurden und kann zusätzlich oder alternativ nach falsch angeschlossenen Leitungen prüfen.

In Schritt 144 wird die medizinische Vorrichtung informiert, dass die Disposables eingebaut wurden.

Die medizinische Vorrichtung führt in Schritt 146 einen Funktionstest der Leitungen durch.

In Schritt 148 informiert die medizinische Vorrichtung das AR-Device, über den positiven Test bzw. das Resultat.

In Schritt 150 wird das Resultat in dem AR-Device angezeigt. Falls das Resultat negativ ist, werden die Schritte ab Schritt 142 wiederholt.

Wenn alles in Ordnung ist, wird in Schritt 152 in dem AR-Device der nächste Schritt angezeigt.

## Patentansprüche

1. Verfahren zur Aktivierung oder Durchführung eines Funktionstests einer Blutbehandlungsvorrichtung (8) aufweisend die Schritte:
• Erfassen eines Ist-Zustandes der Blutbehandlungsvorrichtung (8) mittels einer Kamera (4), welche in/an einer Augmented-Reality-Vorrichtung vorgesehen bzw. angebracht ist,
• Vergleichen des Ist-Zustandes der Blutbehandlungsvorrichtung (8) mit einem Soll-Zustand der Blutbehandlungsvorrichtung (8) durch eine Steuereinheit (CPU), die mit der Kamera (4) in Verbindung steht,
• Senden eines Befehls für den Start des Funktionstests von der Steuereinheit (CPU) an die Blutbehandlungsvorrichtung (8) auf Basis des Vergleichs,
• Aktivieren des Funktionstests der Blutbehandlungsvorrichtung (8) aufgrund des Befehls.

2. Verfahren nach Anspruch 1, gekennzeichnet ferner durch die Schritte:
• Senden eines Funktionstestergebnisses an die Steuereinheit (CPU),
• Anzeigen des Funktionstestergebnisses in der Augmented-Reality-Vorrichtung (2).

3. Verfahren nach Anspruch 1 oder 2, gekennzeichnet ferner durch den Schritt:
• Einblenden von noch erforderlichen durchzuführenden Schritten zum Erreichen des Soll-Zustandes der Blutbehandlungsvorrichtung (8) in der Augmented-Reality-Vorrichtung (2).

4. Verfahren nach einem der Ansprüche 1 bis 3, gekennzeichnet ferner durch den Schritt:
• Sperren weiterer Funktionstests bei einem negativen Funktionstestergebnis.

5. Verfahren nach einem der Ansprüche 1 bis 4, **dadurch gekennzeichnet, dass** der Funktionstest ein Selbsttest der Blutbehandlungsvorrichtung (8) zum Überprüfen von von einem Nutzer vorgenommenen Aufrüstungshandlungen bei einer Aufrüstung der Blutbehandlungsvorrichtung (8) ist.

6. Verfahren nach einem der Ansprüche 1 bis 5, **dadurch gekennzeichnet, dass** der Funktionstest ein Drucktest ist, der einen zu Testzwecken temporär aufgebauten Druck in Einmalprodukten, welche an/in der Blutbehandlungsvorrichtung (8) angebracht bzw. vorgesehen sind, misst.

7. System aufweisend wenigstens eine Kamera (4), wenigstens eine Steuereinheit (CPU), die in datentechnischer Verbindung mit der Kamera (4) steht oder bringbar ist, und wenigstens eine Blutbehandlungsvorrichtung (8), die wiederum in datentechnischer Verbindung mit der Steuereinheit (CPU) steht oder bringbar ist, **dadurch gekennzeichnet, dass** die Steuereinheit (CPU) eingerichtet ist,
• einen Ist-Zustand der Blutbehandlungsvorrichtung (8) mittels der Kamera (4), die in/an einer Augmented-Reality-Vorrichtung (2) vorgesehen bzw. angeordnet ist, zu erfassen,
• den Ist-Zustand der Blutbehandlungsvorrichtung (8) mit einem Soll-Zustand der Blutbehandlungsvorrichtung (8) zu vergleichen,
• einen Befehl für den Start eines Funktionstests an die Blutbehandlungsvorrichtung (8) auf der Basis des Vergleichs zu senden, und
• den Funktionstest der Blutbehandlungsvorrichtung (8) aufgrund des Befehls zu aktivieren.

8. System nach Anspruch 7, **dadurch gekennzeichnet, dass** die Kamera (4) an/in der Augmented-Reality-Vorrichtung (2) angebracht bzw. vorgesehen ist, wobei die Augmented-Reality-Vorrichtung (2) eingerichtet ist, ein Funktionstestergebnis und/oder weitere von einem Anwender durchzuführende Schritte darzustellen.

9. System nach Anspruch 7 oder 8, **dadurch gekennzeichnet, dass** der Funktionstest ein Selbsttest der Blutbehandlungsvorrichtung (8) zum Überprüfen von von einem Nutzer vorgenommenen Aufrüstungshandlungen bei einer Aufrüstung der Blutbehandlungsvorrichtung (8) ist.

10. System nach einem der Ansprüche 7 bis 9, **dadurch gekennzeichnet, dass** der Funktionstest ein Drucktest ist, der einen zu Testzwecken temporär aufgebauten Druck in Einmalprodukten, welche an/in der Blutbehandlungsvorrichtung (8) angebracht bzw. vorgesehen sind, misst.

## Claims

1. A method for activating or performing a function test of a blood treatment device (8), comprising the following steps:
• detecting an actual state of the blood treatment device (8) by means of a camera (4), which is provided or attached in/to an augmented reality device,
• comparing the actual state of the blood treatment device (8) with a target state of the blood treatment device (8) by means of a control unit (CPU) connected to the camera (4),
• transmitting, on the basis of the comparison, a command for starting the function test to the blood treatment device (8) by the control unit (CPU),
• activating the function test of the blood treatment device (8) on the basis of the command.

2. The method according to claim 1, further **characterized by** the steps of:
• transmitting a function test result to the control unit (CPU),
• displaying the function test result in the augmented reality device (2).

3. The method according to claim 1 or 2, further **characterized by** the step of:
• overlaying, in the augmented reality device (2), of still necessary steps to be performed in order to achieve the target state of the blood treatment device (8).

4. The method according to one of claims 1 to 3, further **characterized by** the step of:
• blocking further function tests in the case of a negative function test result.

5. The method according to one of claims 1 to 4, **characterized in that** the function test is a self-test of the blood treatment device (8) for checking setting-up actions performed by a user during a set-up of the blood treatment device (8).

6. The method according to one of claims 1 to 5, **characterized in that** the function test is a pressure test measuring a pressure temporarily built up for test purposes in disposable products, which are attached or provided to/in the blood treatment device (8).

7. A system comprising at least one camera (4), at least one control unit (CPU) which is or can be brought in data connection with the camera (4), and at least one blood treatment device (8), which in turn is or can be brought in data connection with the control unit (CPU), **characterized in that** the control unit (CPU) is configured
• to detect an actual state of the blood treatment device (8) by means of the camera (4), which is provided or arranged in/on an augmented reality device (2),
• to compare the actual state of the blood treatment device (8) with a target state of the blood treatment device (8),
• to transmit, on the basis of the comparison, a command for starting a function test to the blood treatment device (8), and
• to activate the function test of the blood treatment device (8) on the basis of the command.

8. The system according to claim 7, **characterized in that** the camera (4) is attached or provided to/in the augmented reality device (2), the augmented reality device (2) being configured to display a function test result and/or further steps to be performed by a user.

9. The system according to claim 7 or 8, **characterized in that** the function test is a self-test of the blood treatment device (8) for checking setting-up actions performed by a user when setting-up the blood treatment device (8).

10. The system according to one of claims 7 to 9, **characterized in that** the function test is a pressure test measuring a pressure temporarily built up for test purposes in disposable products, which are attached or provided to/in the blood treatment device (8).

## Revendications

1. Procédé d'activation ou de réalisation d'un test de fonctionnement d'un dispositif de traitement du sang (8), présentant les étapes consistant à :
- détecter un état effectif du dispositif de traitement du sang (8) au moyen d'une caméra (4) qui est prévue ou disposée dans/sur un dispositif de réalité augmentée,
- comparer l'état effectif du dispositif de traitement du sang (8) avec un état de consigne du dispositif de traitement du sang (8) par l'intermédiaire d'un dispositif de commande (CPU) qui est connecté à la caméra (4),
- envoyer une commande pour le démarrage du test de fonctionnement du dispositif de commande (CPU) au dispositif de traitement de sang (8) sur la base de la comparaison,
- activer le test de fonctionnement du dispositif de traitement du sang (8) sur la base de la commande.

2. Procédé selon la revendication 1, **caractérisé en outre par** les étapes consistant à :
- envoyer un résultat de test de fonctionnement au dispositif de commande (CPU),
- afficher le résultat de test de fonctionnement dans le dispositif de réalité augmentée (2).

3. Procédé selon la revendication 1 ou 2, **caractérisé en outre par** l'étape consistant à :
- afficher dans le dispositif de réalité augmentée (2) des étapes à réaliser encore nécessaires pour atteindre l'état de consigne du dispositif de traitement du sang (8).

4. Procédé selon l'une quelconque des revendications 1 à 3, **caractérisé en outre par** l'étape consistant à :
- bloquer des tests de fonctionnement supplémentaires si le résultat de test de fonctionnement est négatif.

5. Procédé selon l'une quelconque des revendications 1 à 4, **caractérisé en ce que** le test de fonctionnement est un autotest du dispositif de traitement du sang (8) pour vérifier les actions de mise à niveau effectuées par un utilisateur lors d'une mise à niveau du dispositif de traitement du sang (8).

6. Procédé selon l'une quelconque des revendications 1 à 5, **caractérisé en ce que** le test de fonctionnement est un test de pression qui mesure une pression établie temporairement à des fins de test dans des produits à usage unique qui sont disposés ou prévus sur/dans le dispositif de traitement du sang (8).

7. Système présentant au moins une caméra (4), au moins un dispositif de commande (CPU) qui est ou peut être mis en communication de données avec la caméra (4) et au moins un dispositif de traitement du sang (8) qui à son tour est ou peut être mis en communication de données avec le dispositif de commande (CPU), **caractérisé en ce que** le dispositif de commande (CPU) est configuré pour
- détecter un état effectif du dispositif de traitement du sang (8) au moyen de la caméra (4) qui est prévue ou disposée dans/sur un dispositif de réalité augmentée (2),
- comparer l'état effectif du dispositif de traitement du sang (8) avec un état de consigne du dispositif de traitement du sang (8),
- envoyer une commande pour le démarrage d'un test de fonctionnement au dispositif de traitement du sang (8) sur la base de la comparaison, et
- activer le test de fonctionnement du dispositif de traitement du sang (8) sur la base de la commande.

8. Système selon la revendication 7, **caractérisé en ce que** la caméra (4) est disposée ou prévue sur/dans le dispositif de réalité augmentée (2), dans lequel le dispositif de réalité augmentée (2) est configuré pour représenter un résultat de test de fonctionnement et/ou des étapes supplémentaires à effectuer par un utilisateur.

9. Système selon la revendication 7 ou 8, **caractérisé en ce que** le test de fonctionnement est un autotest du dispositif de traitement du sang (8) pour vérifier des actions de mise à niveau effectuées par un utilisateur lors d'une mise à niveau du dispositif de traitement du sang (8).

10. Système selon l'une quelconque des revendications 7 à 9, **caractérisé en ce que** le test de fonctionnement est un test de pression qui mesure une pression établie temporairement à des fins de test dans des produits à usage unique qui sont disposés ou prévus sur/dans le dispositif de traitement du sang (8).
